# FASCICULE DE BREVET EUROPEEN

(11) **EP 1 857 142 B1**
(45) Date de publication et mention de la délivrance du brevet: **26.03.2008**
(21) Numéro de dépôt: 07290605.0
(22) Date de dépôt: 14.05.2007
(51) Int. Cl.: A61N 1/365, A61N 1/37

(54) **Dispositif médical implantable actif de stimulation cardiaque, resynchronisation, cardioversion et/ou défibrillation, comportant des moyens de détection d'artefacts de bruit ventriculaire**
Aktives medizinisches Implantat zur Herzstimulation, Resynchronisation, Kardioversion und/oder Defibrillation, das Mittel zum Erfassen von Ventrikelgeräuschartefakten umfasst
Active implantable medical device for cardiac stimulation, resynchronisation, cardioversion and/or defibrillation, comprising means for detecting ventricular noise artefacts

(30) Priorité: 18.05.2006 FR 0604446
(43) Date de publication de la demande: 21.11.2007
(73) Titulaire: ELA MEDICAL, 92541 Montrouge (FR)
(72) Inventeur: Limousin, Marcel, 75014 Paris (FR); Vincent, Elodie, 92160 Antony (FR)
(74) Mandataire: Dupuis-Latour, Dominique

(56) Documents cités:
- EP-A- 1 433 496
- EP-A- 1 533 001
- EP-A- 1 537 894
- EP-A1- 0 653 225
- EP-A2- 0 655 260
- WO-A-20/05018738
- US-A- 5 647 379
- US-A- 5 776 168
- US-B2- 6 892 092

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation, de cardioversion et/ou de défibrillation en cas de trouble du rythme détecté par le dispositif.

L'analyse du rythme cardiaque est effectuée à partir de signaux d'électro-gramme (EGM), recueillis par des électrodes portées par des sondes endocavitaires implantées dans le myocarde pour mesurer le potentiel de dépolarisation auriculaire et/ou ventriculaire. Ces signaux sont analysés par l'implant, qui délivrera éventuellement au patient une thérapie appropriée sous forme d'impulsions de faible énergie (stimulation antibradycardique ou stimulation de resynchronisation des ventricules) ou de chocs de cardioversion ou de défibrillation.

L'analyse du rythme, et donc la décision de délivrer ou non une thérapie, peut être cependant perturbée par des artefacts recueillis par la sonde endocavitaire.

Ces artefacts peuvent être d'origines diverses. Une première série d'artefacts correspond à des situations où le dispositif détecte non seulement l'évènement proprement dit, c'est-à-dire l'onde de dépolarisation de la cavité considérée, mais également une perturbation associée à ce même événement et considérée, à tort, comme un autre événement survenu après le premier : onde de dépolarisation tardive, diaphonie entre cavités,...

Une autre série d'artefacts, qui sont ceux concernés par la présente invention, sont les artefacts de bruit extrinsèque, non lié à la dépolarisation du myocarde. Ce bruit peut avoir plusieurs origines, dont notamment les myopotentiels associés aux contractions musculaires, ainsi que les interférences électromagnétiques provenant d'équipements électroniques de surveillance, d'appareils électriques environnants, d'instruments électro-chirurgicaux, de systèmes de communication, etc.

Ce bruit, s'il est présent avec plus ou moins de régularité, peut alors être détecté par l'implant comme une dépolarisation du myocarde, avec le risque de générer des thérapies inappropriées, par exemple en inhibant à tort les stimulations antibradycardiques ou les thérapies de resynchronisation ou, inversement, en délivrant à tort des chocs inappropriés.

Diverses techniques ont été proposées pour réduire l'incidence de ces bruits extrinsèques, notamment l'application d'un filtrage analogique ou numérique, l'instauration de périodes réfractaires, l'adaptation automatique de sensibilité des amplificateurs de détection, ou le contrôle automatique du gain de ces amplificateurs.

Cependant, l'utilisation de ces divers moyens se fait toujours au détriment d'une bonne détection.

En particulier, afin d'assurer la détection de la fibrillation ventriculaire (FV) dont le niveau du signal est faible, il est nécessaire de rechercher une sensibilité maximale de détection, sous peine de ne pas détecter des évènements qui auraient dû l'être. En effet, l'amplitude des signaux de fibrillation ventriculaire peut se situer à un niveau variable compris entre le niveau des signaux de bruit susceptibles d'être détectés par la prothèse, et celui des signaux des complexes sinusaux. Si l'on veut pouvoir détecter la fibrillation ventriculaire, la détection d'un bruit éventuel est donc inévitable. Si, de plus, l'on se trouve en présence d'un bruit régulier, pour un patient présentant un rythme cardiaque sinusal normal ce bruit peut être confondu avec des dépolarisations. Cette situation peut fausser l'évaluation du rythme moyen par l'implant, ce rythme étant estimé à un niveau très supérieur à la réalité, avec un risque corrélatif d'application d'une thérapie anti-tachycardique indésirable (faux positif). Inversement, si l'appareil est programmé à une valeur de sensibilité trop faible, c'est-à-dire avec un seuil de sensibilité trop élevé, les épisodes réels de fibrillation ventriculaire risquent de ne pas être détectés (faux négatif), avec des conséquences encore plus graves pour le patient.

La détection du bruit extrinsèque étant généralement inévitable, le problème de l'invention consiste à discriminer ce bruit parmi les dépolarisations cardiaques afin d'éviter le déclenchement de traitements inappropriés causés par ce bruit d'origine externe.

Le point de départ de l'invention est la constatation de ce que la dépolarisation, qui est un phénomène électrique sensible au bruit, est normalement suivie d'une contraction cardiaque, qui est un phénomène mécanique qui n'est pas affecté par le bruit. De la sorte, en procédant à une double détection - de la dépolarisation et de la contraction - par des moyens distincts, on peut en présence de bruit suspect, opérer un lever de doute pour confirmer que le signal détecté a été effectivement suivi par une activité mécanique du coeur et constitue donc bien un signal de dépolarisation et non un artefact.

La détection de l'activité mécanique du coeur peut en particulier être opérée par mesure de l'accélération endocardiaque, réalisée par un accéléromètre directement en contact avec le muscle cardiaque (généralement à l'apex ventriculaire droit).

On sait en effet que l'accélération endocardiaque reflète très précisément et en temps réel les phénomènes concourant au fonctionnement mécanique du coeur.

Plus précisément, le EP-A-0 515 319 (Sorin Biomedica Cardio SpA) enseigne la manière de recueillir un signal d'accélération endocardiaque au moyen d'une sonde endocavitaire pourvue d'une électrode distale de stimulation implantée au fond du ventricule et intégrant un micro-accéléromètre permettant de mesurer l'accélération endocardiaque.

Le signal d'accélération endocardiaque ainsi recueilli au cours d'un cycle cardiaque forme notamment deux pics correspondant aux deux bruits majeurs qu'il est possible de reconnaître dans chaque cycle d'un coeur sain :
- le premier pic d'accélération endocardiaque ("PEA I") correspond à la fermeture des valvules mitrale et tricuspide, au début de la phase de contraction ventriculaire isovolumique (systole). Les variations de ce premier pic sont étroitement liés aux variations de la pression dans le ventricule (l'amplitude du pic PEA I étant, plus précisément, corrélée au maximum positif de la variation de pression dP/dt dans le ventricule gauche) et peuvent donc constituer un paramètre représentatif de la contractilité du myocarde.
- le second pic d'accélération endocardiaque ("PEA II"), quant à lui, correspond à la fermeture des valvules aortique et pulmonaire, au début de la diastole. Il est produit par la décélération brusque de la masse sanguine en mouvement dans l'aorte.

Le EP-A-0 655 260 (Sorin Biomedica Cardio SpA) décrit une manière de traiter le signal d'accélération endocavitaire délivré par le capteur situé en bout de sonde pour en dériver deux valeurs respectives liées à ces pics d'accélération endocardiaque.

Dans ce document, il est proposé d'utiliser les valeurs d'amplitude des pics PEA I et PEA II pour détecter des troubles cardiaques et déclencher ou non une thérapie de défibrillation.

Dans le cas de la présente invention, il s'agit de détecter la présence ou l'absence d'une contraction cardiaque, en partant du principe qu'à chaque cycle cardiaque réel correspond une seule contraction cardiaque. L'accélération endocardiaque est analysée, avantageusement en détectant la présence ou non d'un pic PEA I, pour confirmer la présence d'une activité mécanique du coeur sur détection d'une dépolarisation : une telle détection qui ne serait pas suivie par une activité mécanique du coeur peut avoir été générée par du bruit, elle est donc suspecte et ne doit pas entraîner systématiquement l'application ou l'inhibition (selon le cas) d'une thérapie.

Le dispositif de l'invention est du type décrit par le EP-A-0 655 260 précité, correspondant au préambule de la revendication 1, et il comprend en outre les moyens énoncés par la partie caractéristique de cette revendication 1. Les sous-revendications visent des formes de mise en oeuvre avantageuses.

On va maintenant décrire un exemple de mise en oeuvre du dispositif de l'invention, en référence aux dessins annexés.
La figure 1 est un chronogramme montrant, au cours de trois cycles cardiaques successifs, les variations de l'accélération endocavitaire ainsi que de l'électrogramme et de l'électrocardiogramme de surface.
La figure 2 est un autre chronogramme montrant, au cours de six cycles cardiaques successifs : (i) les différents signaux recueillis représentatifs de dépolarisations successives, (ii) les signaux indiquant la présence d'un pic d'accélération endocardiaque, et (iii) les modifications apportées à la sensibilité de détection en présence de bruit.
La figure 3 est un organigramme illustrant la succession des différentes étapes d'analyse pour la mise en oeuvre de l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque ou défibrillateur/cardioverteur, comprenant des moyens d'acquisition d'un signal fourni par des sondes endocavitaires et/ou un ou plusieurs capteurs implantés.

L'invention peut notamment être appliquée aux dispositifs implantables commercialisés par ELA Médical, Montrouge, France tels que les appareils *Symphony et ELA Rhapsody.*

Il s'agit de dispositifs à microprocesseur programmables comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

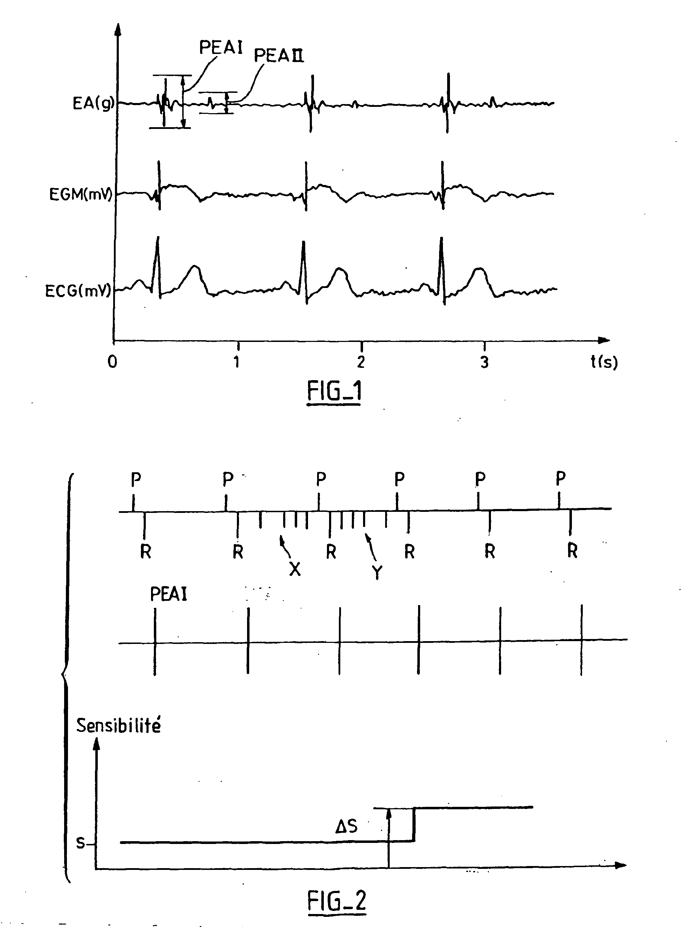

Sur la figure 1, on a représenté (tracé du haut), les variations de l'accélération endocardiaque (EA), mesurée par un capteur tel que celui décrit dans le EP-A-0 515 319 précité, intégré à une tête de sonde endocavitaire placée au fond du ventricule. On a également illustré sur cette figure les tracés de l'électrogramme (EGM), c'est-à-dire du signal électrique recueilli par l'électrode distale de ce même capteur, et d'un électrocardiogramme de surface (ECG) correspondant, au cours de trois cycles cardiaques consécutifs. Comme on l'a expliqué plus haut le tracé de l'accélération présente deux complexes successifs ou pics d'accélération endocardiaque (PEA), dont les paramètres (amplitude, durée et position temporelle, c'est-à-dire instant de survenue) peuvent être déterminées par un traitement approprié du signal délivré par le capteur d'accélération, comme décrit dans le EP-A-0 655 260 précité.

La présente invention propose d'utiliser les paramètres liés à l'accélération endocardiaque ainsi recueillis, notamment la survenue du pic PEA I (indiquée par la position temporelle de ce pic), pour confirmer ou infirmer la présence d'une activité mécanique du coeur.

La première ligne de la figure 2 illustre la succession des évènements auriculaires P et ventriculaires R au cours de six cycles cardiaques successifs, pour un patient présentant un rythme sinusal normal.

Le recueil de ces signaux peut être perturbé par la détection de bruits ventriculaires extrinsèques qui peuvent se traduire par des artefacts, tels que ceux illustrés en X et Y, susceptibles d'être interprétés (à tort) par le dispositif comme des évènements ventriculaires conduisant à une suspicion erronée d'augmentation brutale du rythme ventriculaire, similaire à ce qui pourrait apparaître en cas de fibrillation ventriculaire.

En revanche, la séquence des pics d'accélération (seconde ligne de la figure 2) n'est pas perturbée par du bruit, puisqu'il s'agit de la détection d'une activité purement mécanique, comme on l'a expliqué plus haut.

Le caractère régulier des contractions permet d'écarter la suspicion de fibrillation ventriculaire et de qualifier d'artefacts les évènements suspects X et Y.

Pour éviter la détection de ce bruit ventriculaire sur les cycles cardiaques suivants, il est avantageux de moduler la sensibilité des circuits de détection des dépolarisations, en augmentant le seuil de détection S d'un incrément ΔS (troisième ligne de la figure 2), pendant une durée prédéterminée, ou pendant un nombre de cycles prédéterminé. Cette réduction de sensibilité (incrément ΔS) peut être éventuellement modulée en fonction du niveau du bruit détecté.

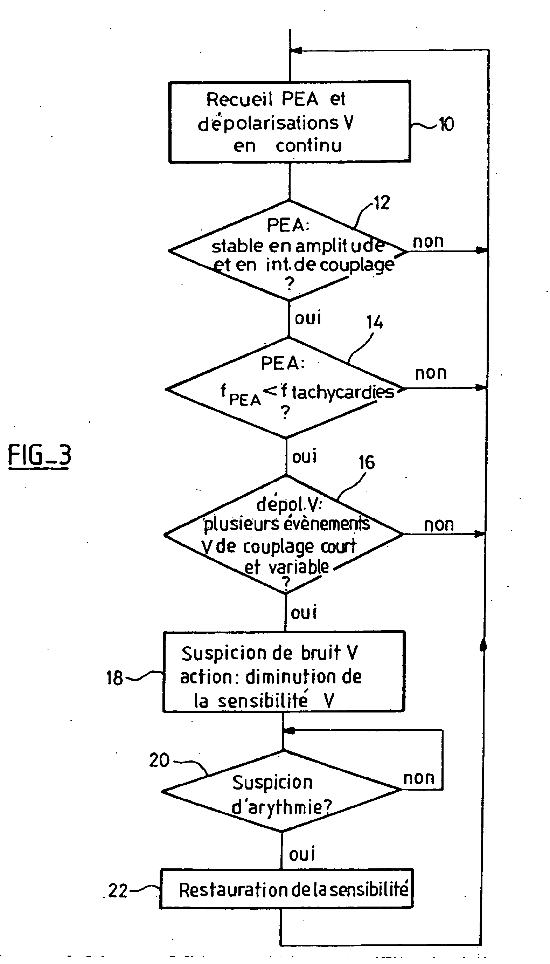

On va maintenant décrire plus en détail, en référence à l'organigramme de la figure 3, la manière dont est opérée la corrélation entre les signaux représentatifs des dépolarisations (première ligne de la figure 2) et ceux représentatifs des pics d'accélération (seconde ligne de la figure 2)

La première étape, référencée 10, consiste à recueillir de façon continue les signaux d'accélération endocardiaque et les dépolarisations ventriculaires, l'analyse étant opérée sur chaque cycle cardiaque.

Le dispositif détermine à partir de ces mesures une première série de signaux représentatifs des dépolarisations ventriculaires, et une deuxième série de signaux représentatifs des pics d'accélération (avantageusement le pic PEA I).

La première phase de l'analyse (étape 12) consiste à déterminer si les signaux de pics PEA sont stables en amplitude et/ou en intervalles de couplage (l'intervalle de couplage étant la période temporelle séparant deux pics relatifs à des cycles consécutifs). La condition de stabilité en amplitude signifie par exemple que l'amplitude du pic PEA I ne varie pas de plus de x % par rapport à la moyenne des y cycles précédents. La condition de stabilité du couplage signifie que l'intervalle de couplage ne varie pas de plus ou moins z millisecondes, par exemple plus ou moins 30 millisecondes d'un cycle au suivant.

En présence d'un rythme PEA stable révélateur de contractions régulières, le dispositif détermine (étape 14) si la fréquence de ces contractions (fréquence des pics PEA) est inférieure à une fréquence limite, inférieure à la zone de détection des tachycardies.

Dans la négative, il s'agit vraisemblablement d'une tachycardie avérée, pour laquelle un thérapie doit être envisagée sans qu'il y ait lieu de poursuivre l'analyse.

Dans l'affirmative, donc en présence d'un rythme des contractions suffisamment lent, le dispositif examine (étape 16) s'il est en présence d'une série d'évènements ventriculaires de couplage court et variable (le critère de "couplage court" signifiant que les intervalles de couplage entre événements ventriculaires successifs sont inférieurs à un seuil donné, et le critère de "couplage variable" signifiant que les différences entre les intervalles de couplage dépassent un seuil donné sur un nombre prédéterminé de cycles successifs).

Si l'analyse des dépolarisations ventriculaires révèle à l'étape 16 un rythme rapide et instable, il y a alors suspicion de bruit ventriculaire (étape 18).

Afin d'éviter la détection de ce bruit ventriculaire sur les cycles suivants, la sensibilité du circuit de détection ventriculaire est diminuée (c'est-à-dire que le seuil de sensibilité est relevé) pendant un temps prédéterminé (temps défini en durée ou en nombre de cycles). On notera que d'autres paramètres des circuits de détection peuvent être modifiés en cas de suspicion de bruit ventriculaire, notamment les réglages des circuits ou des algorithmes de filtrage.

II y aura toutefois lieu de restaurer au plus vite la sensibilité ventriculaire à sa valeur initiale en cas de suspicion d'arythmie ou de perte du signal sinusal (étapes 20, 22).

La détection des arythmies peut être notamment mise en oeuvre par l'algorithme mis en oeuvre par l'algorithme "PARAD" (marque déposée d'ELA Medical), qui est un algorithme de diagnostic décrit notamment dans les EP-A-0 626 182 et EP-A-0 838 235 (ELA Medical), auxquels on pourra se référer pour plus de détails.

Elle peut être également mise en oeuvre par détection d'une accélération des pics de contraction cardiaque : par exemple une accélération de 25 % de la moyenne des 8 intervalles précédents sera considérée comme une situation correspondant à une suspicion d'arythmie.

Par ailleurs, dans ce qui précède on a décrit un dispositif utilisant les signaux d'accélération endocardiaque au niveau du ventricule droit. Mais cette caractéristique n'est pas limitative, et l'invention peut être également mise en oeuvre en utilisant des signaux représentatifs de l'accélération endocardiaque relevée au niveau :
- d'une oreillette,
- ou du ventricule gauche,
- ou d'un vaisseau sanguin périphérique du coeur, c'est-à-dire d'un vaisseau situé sur le coeur ou à proximité immédiate du coeur (en contact avec la paroi du coeur).

## Revendications

1. Un dispositif médical implantable actif du type prothèse cardiaque de stimulation, resynchronisation, cardioversion et/ou défibrillation, comporta nt :
- des moyens de détection du rythme comprenant au moins une électrode endocavitaire apte à recueillir des potentiels électriques représentatifs de dépolarisations du myocarde, et un circuit de détection apte à analyser les potentiels recueillis et délivrer une séquence de signaux représentatifs des dépolarisations ventriculaires et auriculaires (P, R) successives, et
- des moyens de détection des contractions du myocarde comprenant un capteur d'accélération endocardiaque, et des moyens pour déterminer au moins un pic de l'accélération endocardiaque au cours d'un cycle cardiaque donné et délivrer une séquence de signaux représentatifs des pics d'accélération (PEA I) successifs,
dispositif **caractérisé en ce qu**'il comporte en outre des moyens de recherche d'artefacts de bruit ventriculaire (X, Y), comprenant des moyens pour :
- recevoir en entrée et corréler entre eux lesdits signaux représentatifs des dépolarisations et lesdits signaux représentatifs des pics d'accélération, et
- en cas de défaut de corrélation, délivrer un signal de suspicion de bruit ventriculaire.

2. Le dispositif de la revendication 1, comprenant en outre des moyens pour modifier un paramètre de fonctionnement des moyens de détection du rythme, en réponse à la délivrance d'un signal de suspicion de bruit ventriculaire.

3. Le dispositif de la revendication 2, dans lequel ledit paramètre de fonctionnement est un seuil de sensibilité (S) dudit circuit de détection, et les moyens pour modifier le paramètre de fonctionnement comportent des moyens pour élever ce seuil (ΔS), en réponse à la délivrance d'un signal de suspicion de bruit ventriculaire.

4. Le dispositif de la revendication 2, dans lequel le circuit de détection comporte un filtre numérique et ledit paramètre de fonctionnement est un paramètre de ce filtre numérique, et dans lequel les moyens pour modifier le paramètre de fonctionnement comportent des moyens pour modifier le paramètre du filtre dans le sens d'un filtrage plus restrictif, en réponse à la délivrance d'un signal de suspicion de bruit ventriculaire.

5. Le dispositif de la revendication 2, dans lequel les moyens pour modifier le paramètre de fonctionnement comportent des moyens pour modifier ce paramètre pendant une durée prédéterminée, ou pendant un nombre de cycles prédéterminé, suivant la délivrance du signal de suspicion de bruit ventriculaire.

6. Le dispositif de la revendication 1, dans lequel le dispositif comprend des moyens d'analyse desdits signaux représentatifs des dépolarisations ventriculaires et auriculaires successives, et dans lequel les moyens pour modifier le paramètre de fonctionnement comportent des moyens (20, 22) pour restaurer le paramètre à sa valeur antérieure en cas de suspicion d'arythmie.

7. Le dispositif de la revendication 6, dans lequel les moyens (20, 22) pour restaurer le paramètre à sa valeur antérieure en cas de suspicion d'arythmie comprennent des moyens d'analyse des dépolarisations ventriculaires et auriculaires successives.

8. Le dispositif de la revendication 7, dans lequel les moyens (20, 22) pour restaurer le paramètre à sa valeur antérieure en cas de suspicion d'arythmie comprennent des moyens de détection d'une accélération des pics de contraction cardiaque.

9. Le dispositif de la revendication 1, comprenant deux sondes endocavitaires distinctes, l'une portant ladite électrode endocavitaire, l'autre portant ledit capteur d'accélération endocardiaque.

10. Le dispositif de la revendication 1, dans lequel la délivrance d'un signal de suspicion de bruit ventriculaire (18) par les moyens de recherche d'artefacts est conditionnée par la détection (12) d'une séquence de pics d'accélération stable en amplitude et/ou en intervalles de couplage.

11. Le dispositif de la revendication 1, dans lequel la délivrance d'un signal de suspicion de bruit ventriculaire (18) par les moyens de recherche d'artefacts est conditionnée par la détection (14) d'une séquence de pics d'accélération de fréquence inférieure à une fréquence limite représentative d'un seuil de détection des tachycardies.

12. Le dispositif de la revendication 1, dans lequel la délivrance d'un signal de suspicion de bruit ventriculaire (18) par les moyens de recherche d'artefacts est conditionnée par la détection (16) d'une séquence de dépolarisations présentant des intervalles de couplage successifs courts et variables.

13. Le dispositif de la revendication 1, dans lequel le capteur d'accélération est un capteur apte à relever l'accélération au niveau d'un ventricule du coeur.

14. Le dispositif de la revendication 1, dans lequel le capteur d'accélération est un capteur apte à relever l'accélération au niveau d'une oreillette du coeur.

15. Le dispositif de la revendication 1, dans lequel le capteur d'accélération est un capteur apte à relever l'accélération au niveau d'un vaisseau sanguin périphérique du coeur.

## Claims

1. An active implantable medical device of the cardiac prosthesis type for pacing, resynchronization, cardioversion and/or defibrillation, comprising:
- rhythm sensing means comprising at least one endocardial electrode adapted to collect electrical potentials representative of myocardium depolarisations, and a sensing circuit adapted to analyse the collected potentials and issue a sequence of signals that are representative of the successive ventricular and atrial depolarisations (P, R); and
- means for detecting myocardium contractions, comprising an endocardial acceleration sensor, and means for determining at least one peak of endocardial acceleration over one given cardiac cycle and issue a sequence of signals that are representative of the successive endocardial acceleration peaks (PEA I),
said device being **characterised by** further comprising means for searching for ventricular noise artifacts (X, Y), comprising means for:
- receiving as input, and correlating together, said signals representative of the cardiac depolarisations and said signals representative of the acceleration peaks, and
- in case of non-correlation, issuing a signal of suspicion of ventricular noise.

2. The device of claim 1, further comprising means for modifying an operating parameter of said rhythm sensing means, in response to the issuance of a ventricular noise suspicion signal.

3. The device of claim 2, wherein said operating parameter is a sensing sensitivity threshold (S) of said sensing circuit, and the means for modifying the operating parameter comprise means for increasing said threshold (ΔS) in response to the issuance of a ventricular noise suspicion signal.

4. The device of claim 2, wherein the sensing circuit further comprises a digital filter and said operating parameter is a parameter of said digital filter, and wherein the means for modifying the operating parameter comprise means for modifying the filter parameter towards a more restrictive filtering in response to the issuance of a ventricular noise suspicion signal.

5. The device of claim 2, wherein the means for modifying the operating parameter comprises means for modifying said parameter for a predetermined duration, or during a predetermined number of cycles, following the issuance of the ventricular noise suspicion signal.

6. The device of claim 1, wherein the device comprises means for analysing said signals representative of the successive ventricular and atrial depolarisations, and wherein the means for modifying the operating parameter comprises means (20, 22) for restoring the operating parameter to its previous value in the event of an arrhythmia suspicion.

7. The device of claim 6, wherein the means (20, 22) for restoring the parameter to its previous value in the event of an arrhythmia suspicion comprises means for analysing the successive ventricular and atrial depolarisations.

8. The device of claim 7, wherein the means (20, 22) for restoring the parameter to its previous value in the event of an arrhythmia suspicion comprises means for detecting an acceleration of the cardiac contraction peaks.

9. The device of claim 1, comprising two distinct endocardial leads, one equipped with said endocardial electrode, the other with said endocardial acceleration sensor.

10. The device of claim 1, wherein the issuance of a ventricular noise suspicion signal (18) through the means for searching for artifacts is conditioned on the detection (12) of a sequence of acceleration peaks that are stable in terms of amplitude and/or coupling intervals.

11. The device of claim 1, wherein the issuance of a ventricular noise suspicion signal (18) through the means for searching for artifacts is conditioned on the detection (14) of a sequence of acceleration peaks with a rate that is lower than a limit rate representative of a threshold of tachycardia detection.

12. The device of claim 1, wherein the issuance of a ventricular noise suspicion signal (18) through the means for searching for artifacts is conditioned on the detection (16) of a sequence of depolarisations showing short and variable successive coupling intervals.

13. The device of claim 1, wherein the acceleration sensor is a sensor adapted to measure the acceleration at a ventricle of the heart.

14. The device of claim 1, wherein the acceleration sensor is a sensor adapted to measure the acceleration at an atrium of the heart.

15. The device of claim 1, wherein the acceleration sensor is a sensor adapted to measure acceleration level at a blood vessel that is peripheral to the heart.

## Patentansprüche

1. Aktive medizinische implantierbare Vorrichtung vom Typ einer Prothese zur Stimulation, Resynchronisation, Kardioversion und/oder Defibrillation des Herzens, aufweisend:
- Mittel zur Rhythmuserfassung, die wenigstens eine endokavitäre Elektrode, die dazu geeignet ist elektrische Potentiale aufzunehmen, die für Depolarisationen des Herzmuskels repräsentativ sind, und einen Erfassungsschaltkreis, der geeignet ist, die aufgenommenen Potentiale zu analysieren und eine Folge an Signalen auszugeben, die für aufeinanderfolgende ventrikuläre und aurikuläre (P, R) Depolarisationen repräsentativ sind, aufweisen, und
- Mittel zur Erfassung der Herzmuskelkontraktionen, die einen Sensor der endokardialen Beschleunigung und Mittel zum Bestimmen wenigstens einer endokardialen Beschleunigungsspitze während eines gegebenen Herzzyklus und zum Erzeugen einer Folge an Signalen, die für aufeinanderfolgende Beschleunigungsspitzen (PEA 1) repräsentativ sind, aufweisen,
wobei die Vorrichtung **dadurch** charakterisiert ist, dass sie darüber hinaus Mittel zur Recherche nach ventrikulären Rauschartefakten (X, Y) aufweist, die Mittel aufweisen zum:
- Empfangen am Eingang und untereinander korrelieren der genannten für Depolarisationen repräsentativen Signale und der genannten für Beschleunigungsspitzen repräsentativen Signale, und
- falls keine Korrelation vorliegt, Erzeugen eines Signals des Verdachts auf ventrikuläres Rauschen

2. Vorrichtung nach Anspruch 1, weiter aufweisend Mittel zum Modifizieren eines Funktionsparameters der Mittel zur Rhythmuserfassung, in Antwort auf die Erzeugung eines Signals des Verdachts auf ventrikuläres Rauschen.

3. Vorrichtung nach Anspruch 2, in welcher der genannte Funktionsparameter eine Sensibilitätsschwelle (S) des genannten Erfassungsschaltkreis ist und die Mittel zum Modifizieren des Funktionsparameters Mittel zum Anheben dieser Schwelle (ΔS), in Antwort auf die Erzeugung eines Signals des Verdachts auf ventrikuläres Rauschen, aufweisen.

4. Vorrichtung nach Anspruch 2, in welcher der Erfassungsschaltkreis ein digitales Filter aufweist und der genannte Funktionsparameter ein Parameter dieses digitalen Filters ist und in welcher die Mittel zum Modifizieren des Funktionsparameters Mittel zum Modifizieren des Parameters des Filters in Richtung einer restriktiveren Filterung, in Antwort auf die Erzeugung eines Signals des Verdachts auf ventrikuläres Rauschen, aufweisen.

5. Vorrichtung nach Anspruch 2, in welcher die Mittel zum Modifizieren des Funktionsparameters Mittel zum Modifizieren dieses Parameters während einer vorbestimmten Dauer oder während einer vorbestimmten Anzahl an Zyklen, die auf die Erzeugung des Signals des Verdachts auf ventrikuläres Rauschen folgen, aufweisen.

6. Vorrichtung nach Anspruch 1, in welcher die Vorrichtung Mittel zur Analyse der genannten für aufeinanderfolgende ventrikuläre und aurikuläre Depolarisationen repräsentativen Signale aufweisen und in welcher die Mittel zum Modifizieren des Funktionsparameters Mittel (20, 22) zum Setzen des Parameters auf seinen vorherigen Wert im Falle des Verdachts auf Rhythmusstörungen, aufweisen.

7. Vorrichtung nach Anspruch 6, in welcher die Mittel (20, 22) zum Setzen des Parameters auf seinen vorherigen Wert im Falle des Verdachts auf Rhythmusstörungen Mittel zur Analyse der aufeinanderfolgenden ventrikulären und aurikulären Depolarisationen aufweisen.

8. Vorrichtung nach Anspruch 7, in welcher die Mittel (20, 22) zum Setzen des Parameters auf seinen vorherigen Wert im Fall des Verdachts auf Rhythmusstörungen Mittel zur Detektion einer Beschleunigung der Herzkontraktionsspitzen aufweisen.

9. Vorrichtung nach Anspruch 1, die zwei verschiedene endokavitäre Sonden aufweist, von denen eine die genannte endokavitäre Elektrode trägt und die andere den genannten Sensor der endokardialen Beschleunigung trägt.

10. Vorrichtung nach Anspruch 1, in welcher die Ausgabe eines Signals des Verdachts auf ventrikuläres Rauschen (18) durch die Mittel zur Recherche nach Artefakten durch die Erfassung (12) einer Folge an Beschleunigungsspitzen, deren Amplitude und/oder Intervalle der Kopplung stabil ist, bedingt ist.

11. Vorrichtung nach Anspruch 1, in welcher die Ausgabe eines Signals des Verdachts auf ventrikuläres Rauschen (18) durch die Mittel zur Recherche nach Artefakten durch die Erfassung (14) einer Folge an Beschleunigungsspitzen mit geringerer Frequenz als eine Grenzfrequenz, die für eine Schwelle der Erfassung von Tachykardien repräsentativ ist, bedingt ist.

12. Vorrichtung nach Anspruch 1, in welcher die Ausgabe eines Signals des Verdachts auf ventrikuläres Rauschen (18) durch die Mittel zur Recherche nach Artefakten durch die Erfassung (16) einer Folge an Depolarisationen, die aufeinanderfolgende kurze und variable Intervalle der Kopplung darstellen, bedingt ist.

13. Vorrichtung nach Anspruch 1, in welcher der Sensor der Beschleunigung ein Sensor ist, der dazu geeignet ist, die Beschleunigung auf dem Niveau eines Herzventrikels zu erfassen.

14. Die Vorrichtung nach Anspruch 1, in welcher der Sensor der Beschleunigung ein Sensor ist, der dazu geeignet ist, die Beschleunigung auf dem Niveau eines Herzvorhofes zu erfassen.

15. Vorrichtung nach Anspruch 1, in welcher der Sensor der Beschleunigung ein Sensor ist, der dazu geeignet ist, die Beschleunigung auf dem Niveau eines Herzrandgefäßes zu erfassen.
